# EUROPÄISCHE PATENTSCHRIFT

(11) **EP 3 080 075 B1**
(45) Veröffentlichungstag und Bekanntmachung des Hinweises auf die Patenterteilung: **10.01.2018**
(21) Anmeldenummer: 14802053.0
(22) Anmeldetag: 21.11.2014
(51) Int. Cl.: C07C 265/04, C07C 209/48, C07C 211/14

(54) **N, N-(BIS-2-AMINOALKYL)-1,2-ALKYLDIAMINDERIVATE**
N,N-(BIS-2-AMINOALKYL)-1,2-ALKYLDIAMIN DERIVATIVES
DÉRIVÉS DE N,N-(BIS-2-AMINOALKYLE)-1,2-ALKYLDIAMINE

(30) Priorität: 11.12.2013 EP 13196613
(43) Veröffentlichungstag der Anmeldung: 19.10.2016
(73) Patentinhaber: BASF SE, 67056 Ludwigshafen am Rhein (DE)
(72) Erfinder: JAEGLI, Stephanie, 68163 Mannheim (DE); CHARRAK, Monika, 67063 Ludwigshafen (DE); PANCHENKO, Alexander, 67071 Ludwigshafen (DE); SCHMIDT, Thomas, 67433 Neustadt (DE); MELDER, Johann-Peter, 67459 Böhl-Iggelheim (DE)
(74) Vertreter: BASF IP Association
(86) Internationale Anmeldenummer: PCT/EP2014/075263
(87) Internationale Veröffentlichungsnummer: WO 2015/086293

(56) Entgegenhaltungen:
- EP-A1- 1 881 957
- EP-A2- 0 382 508
- WO-A1-2008/080755

## Beschreibung

Die vorliegende Erfindung betrifft 2-N, N-(Bis-2-aminoalkyl)-1,2-alkyldiaminderivate der Formel (I) als solche 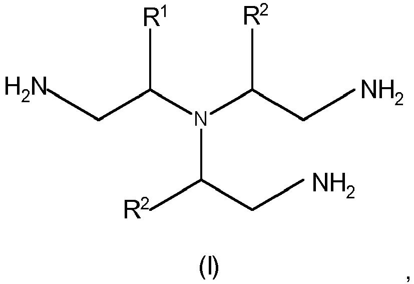 ein Verfahren zur Herstellung der 2-N, N-(Bis-2-aminoalkyl)-1,2-alkyldiaminderivate, die Verwendung der 2-N, N-(Bis-2-aminoalkyl)-1,2-alkyldiaminderivate als Härter für Epoxyharze, als Zwischenprodukt bei der Herstellung von Triisocyanaten, als Starter für Polyetherole und oder als Monomere für die Polyamidherstellung, Triisocyanate abgeleitet aus den 2-N, N-(Bis-2-aminoalkyl)-1,2-alkyldiaminderivate der Formel (I) sowie ein Verfahren zur Herstellung dieser Triisocyanate.

Es ist generell bekannt, dass aliphatische Nitrile in Gegenwart von Wasserstoff und Katalysatoren zu den entsprechenden Aminen hydriert werden können. Solche Hydrierverfahren sind sowohl für β-Aminonitrile als auch für diverse α-Aminonitrile, wie Aminoacetonitril (AAN) oder Ethylendiamindiacetonitril (EDDN), zur Herstellung der entsprechenden Amine, wie Ethylendiamin (EDA) oder Triethylentetramin (TETA), bekannt. Weiterhin ist es bekannt, dass die Hydrierung von β-Aminonitrilen in aller Regel problemlos verläuft, während die Hydrierung von α-Aminonitrilen mit dem Auftreten von zahlreichen Nachteilen verbunden ist, wie der Hydrogenolyse der C-CN-Bindung des eingesetzten Nitrils bzw. der H₂N-C-Bindung des durch die Hydrierung erhaltenen Amins. Das "Handbook of Heterogeneous Catalytic Hydrogenation for Organic Synthesis" (John Wiley & Sons, New York, 2001, Seiten 173-275) zeigt die Problematik der Hydrierung von α-Aminonitril anhand von zyklischen α-Aminonitrilen wie 1-Cyclohexyl-2,5-dicyano-2,5-dimethylpyrrolidin.

EP 382508 beschreibt ein Verfahren zur Hydrierung von Nitrilotriacetonitril (NTAN) als batch-Verfahren. Kontinuierliche Verfahren werden hier explizit ausgeschlossen.

WO 2008/080755 beschreibt ebenfalls die Hydrierung von NTAN. Hierbei wird jedoch in Gegenwart von Ammoniak gearbeitet, da laut WO 2008/080755 nur so primäre Amine erhältlich sind.

In US 8227641 wird die Herstellung von polyfunktionalen Aminen durch hydrieren der entsprechenden polyfunktionalen Nitrilen beschrieben, wobei die Hydrierung in Gegenwart von Alkohol, Wasser oder einer Wasser/Alkoholmischung und Ammoniak durchgeführt wird. WO 2008/104553 betrifft ein Verfahren zur Herstellung von Triethylentetraamin (TETA), wobei Ethylendiamindiacetonitril (EDDN) in Gegenwart eines Katalysators und eines Lösungsmittels hydriert wird. Weiterhin kann EDDN auch als Bestandteil eines Aminonitrilgemisches vorliegen, das zusätzlich Ethylendiaminmonoacetonitril (EDMN) enthält, wobei aus EDMN durch Hydrierung Diethylentriamin (DETA) erhalten wird. Bei TETA und DETA handelt es sich in beiden Fällen um nichtzyklische (lineare) Ethylenamine.

Die im erfindungsgemäßen Verfahren zur Herstellung von 2-N,N-(Bis-2-aminoethyl)-1 ,2-propandiamin (hier als MGTA bezeichnet) eingesetzten entsprechenden Methylglycinenitril N,N-diacetonitril (hier als MGDN bezeichnet) sind bereits aus US 5849950 bekannt. Hier wird die Herstellung von MGDN als solchem beschrieben, wobei alpha-Alaninnitril mit Formaldehyd und Blausäure (HCN) umgesetzt wird. Eine etwaige Hydrierung des bei diesem Verfahren erhaltenen Produkts unter Erhalt der entsprechenden Aminomethylverbindung (MGTA) ist in diesem Dokument jedoch nicht offenbart. Im Stand der Technik sind noch zahlreiche weitere Dokumente bekannt, die allesamt Herstellungsverfahren von MGDN beschreiben, wie beispielsweise EP 1881957. Die Hydrierung von MGDN zu MGTA wird jedoch in keinem der Dokumente beschrieben oder angedeutet.

In EP 375279 wird die Herstellung von Alkylenaminen beschrieben, die durch Ringöffnung von Aziridinringen entstehen. Gemäß der Formel (III) aus EP 375279 würden auch die erfindungsgemäßen 2-N,N-(Bis-2-aminoalkyl)-1,2-alkyldiaminederivate der Formel (I) unter die Formel (III) der EP 375279 fallen, wenn in der Formel (III) R¹=Methyl oder Ethyl, R²=H, R³ und R⁴=C₂₋₄Aminoalkylgruppen und n=1 wäre. Allerdings ist aus Yusin, A. et al. in "Aziridines and Epoxides in Organic Synthesis, ED- Wiley- VCH; Weinheim, Germany 2006 sowie aus Chernitskij K. et al in Zhurnal Obshchei Khimii, 60(3), 617-25; 1990 unter "Nucleophilic cleavage and formation of saturated heterocycles. X. Reactivity of 2-methylaziridine in aminolysis reaction bekannt, dass bei den Ringöffnenden Reaktionen der nukleophile Angriff am sterisch am wenigstens gehindertem C-Atom erfolgt, so dass bei der Formel III von EP 375279 R¹ bevorzugt Wasserstoff ist und R² entsprechend Methyl oder Ethyl. Daher ist aus dem in EP 375279 beschriebenen Verfahren die erfindungsgemäßen 2-N,N-(Bis-2-aminoalkyl)-1,2-diaminoalkylderivaten der Formel (I) nicht erhältlich.

US 3527757 offenbart ebenfalls die Umsetzung von Aziridinringen mit primären oder sekundären Aminen. Die erfindungsgemäßen 2-N,N-(Bis-2-aminoalkyl)-1,2-alkyldiamine der Formel (I) sind über dieses Verfahren jedoch nicht erhältlich da, da zu erwarten ist, dass der Angriff am wenigsten sterisch gehindertem Kohlenstoff stattfindet, so dass das erfindungsgemäße Zielprodukt nicht erhalten wird.

Verbindungen mit zwei oder drei primären Aminofunktionen ("Diamine" oder "Triamine") können für zahlreiche Verwendungen eingesetzt werden, beispielsweise als Härter bei Epoxyharzen oder zur Herstellung von Diisocyanaten oder Triisocyanaten. Die Struktur des eingesetzten Polyamins kann die Eigenschaften der aus den Polyaminen hergestellten Polymermaterialien, wie Wetterbeständigkeit, Hydrolysebeständigkeit, Chemikalienbeständigkeit, Lichtbeständigkeit, elektrische sowie mechanische Eigenschaften, beeinflussen. Sie kann aber auch einen Einfluss auf die Verarbeitbarkeit und die Verarbeitung der Polyamine zu den entsprechenden Polymermaterialien, beispielsweise der Aushärtung von Epoxyharzen, ausüben.

Die Aufgabe der vorliegenden Erfindung ist es daher Verbindungen mit drei primären Aminofunktionen bereit zu stellen, die zur Härtung von Epoxyharzen eingesetzt werden können und neue Eigenschaftsprofile bei der Polyaminherstellung aufweisen. Eine weitere Aufgabe der Erfindung ist es ein Verfahren bereit zu stellen, dass es ermöglicht mit großen Ausbeuten und selektiv 2-N,N-(Bis-2-Aminoalkyl)-1,2-alkyldiamine, die in 2-Position alkyliert sind wie 2-N,N-(Bis-2-aminoethyl)-1,2-propandiamine (MGTA), herzustellen, da über die bekannte Propylenimin Ringöffnung bevorzugt die N,N-(Bis-2-aminoalkyl)-1,2-alkyldiamine erhältlich sind, die maximal in 1-Position alkyliert sind und damit die Regioisomeren der erfindungsgemäßen Verbindungen darstellen.
Des Weiteren soll insbesondere bei der Herstellung von MGTA die Produktion an störenden Nebenprodukten wie 2-Piperazin-1-yl-propan-1-amin verringert werden.

Diese Aufgabe wird gelöst durch 2-N, N-(Bis-2-aminoalkyl)-1,2-alkyldiaminderivate der allgemeinen Formel (I) 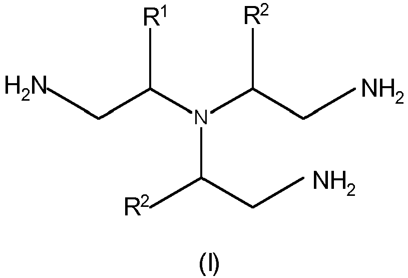 mit
- R¹: ausgewählt aus der Gruppe von verzweigten oder unverzweigten aliphatischen C₁₋₁₀-Alkylgruppen
- R2: ausgewählt aus der Gruppe von Wasserstoff und verzweigten oder unverzweigten aliphatischen C₁₋₁₀-Alkylgruppen.

Vorteilhaft ist es, wenn bei den erfindungsgemäßen Verbindungen der Formel (I) R¹ ausgewählt ist aus der Gruppe von Methyl, Ethyl, Propyl, Isopropyl, n-Butyl, sec.-Butyl und tert. Butyl und R² ausgewählt ist aus der Gruppe von Wasserstoff, Methyl, Ethyl, Propyl, Isopropyl, n-Butyl, sec.-Butyl und tert. Butyl.

Vorteilhaft ist es, wenn bei den erfindungsgemäßen Verbindungen der Formel (I) R¹ Methyl und R² Wasserstoff ist.

Ein weiterer Gegenstand der Erfindung ist ein Verfahren zur Herstellung der Verbindungen der Formel (I) wobei die 2-N, N-(Bis-2-aminoalkyl)-1,2-alkyldiaminderivate aus den entsprechenden 2-[N, N-(Bis-1-cyanoalkyl)amino]alkylnitrilen der Formel (II) 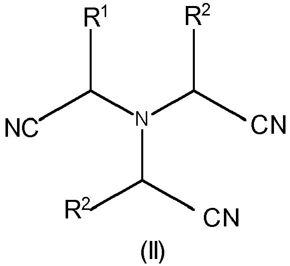 mit
- R¹: ausgewählt aus der Gruppe von verzweigten oder unverzweigten aliphatischen C₁₋₁₀-Alkylgruppen
- R²: ausgewählt aus der Gruppe von Wasserstoff und verzweigten oder unverzweigten aliphatischen C₁₋₁₀-Alkylgruppen
durch Hydrierung in Gegenwart eines Katalysators und Wasserstoff hergestellt werden.

Vorteilhaft ist das erfindungsgemäße Verfahren bei dem R1 ≠ H ist, wobei die Hydierung kontinuierlich durchgeführt wird und unter Ausschluss von Ammoniak gearbeitet wird.

Ein weiterer Gegenstand der Erfindung ist ein Verfahren zur Herstellung der Verbindungen der Formel (I) wobei die 2-N, N-(Bis-2-aminoalkyl)-1,2-alkyldiaminderivate aus den entsprechenden 2-[N,N-(bis-1-cyanoalkyl)amino]alkylnitrilen der Formel (IIa) 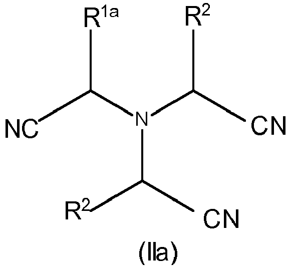 mit
- R^{1a}: ausgewählt aus der Gruppe von verzweigten oder unverzweigten aliphatischen C₁₋₁₀-Alkylgruppen und Wasserstoff
- R²: ausgewählt aus der Gruppe von Wasserstoff und verzweigten oder unverzweigten aliphatischen C₁₋₁₀-Alkylgruppen
durch Hydrierung in Gegenwart eines Katalysators und Wasserstoff hergestellt werden, wobei das Verfahren kontinuierlich und unter Ausschluss von Ammoniak durchgeführt wird.

Vorteilhaft sind die beiden erfindungsgemäßen Verfahren, wenn als Katalysator ein solcher eingesetzt wird der als aktive Spezies ein oder mehrere Elemente ausgewählt aus der Gruppe von Fe, Co, Ni, Ru, Rh, Pd, Os, Ir und Pt enthält.

Vorteilhaft sind die beiden erfindungsgemäßen Verfahren, wenn als Katalysator ein Raney-Nickel oder ein Raney-Kobalt-Katalysator eingesetzt wird.

Vorteilhaft sind die beiden erfindungsgemäßen Verfahren wenn, der eingesetzte Katalysator ein Raney-Kobalt-Katalysator ist, der als Promotor mindestens eins der Elemente Mo, Cr oder Fe enthält.

Vorteilhaft sind die beiden erfindungsgemäßen Verfahren, wenn die Hydrierung in einem Lösungsmittel ausgewählt aus der Gruppe von Amiden, aromatischen Kohlenwasserstoffen, Alkoholen, Aminen, Estern und Ethern durchgeführt wird.

Vorteilhaft sind die beiden erfindungsgemäßen Verfahren, wenn bei Temperaturen im Bereich von 60 bis 180°C und Drücken im Bereich von 40 bis 300 bar hydriert wird.

Vorteilhaft sind die beiden erfindungsgemäßen Verfahren, wenn Methylglycinnitril-diacetonitril (MGDN) zu N,N-(Bis-2-aminoethyl)-1 ,2-propandiamin (MGTA) hydriert wird.

Vorteilhaft sind die beiden erfindungsgemäßen Verfahren, wenn Methylglycinnitril-diacetonitril (MGDN) und NTAN in reiner kristalliner Form eingesetzt wird

Ein weiterer Gegenstand der Erfindung ist die Verwendung eines der 2-N, N-(Bis-2-aminoalkyl)-1,2-alkyldiaminderivate der Formel (I) als Härter für Epoxyharze, als Zwischenprodukt bei der Herstellung von Triisocyanaten, als Starter bei Herstellung von Polyetherolen und/oder als Monomer für die Polyamidherstellung.

Ein weiterer Gegenstand der Erfindung sind Triisocyanate gemäß der allgemeinen Formel (III) 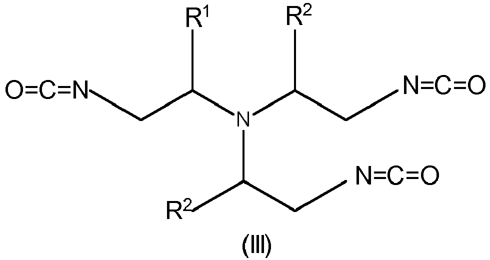 mit
- R¹: ausgewählt aus der Gruppe von verzweigten oder unverzweigten aliphatischen C₁₋₁₀-Alkylgruppen und Wasserstoff
- R²: ausgewählt aus der Gruppe von Wasserstoff und verzweigten oder unverzweigten aliphatischen C₁₋₁₀-Alkylgruppen.

Vorteilhaft ist es, wenn bei den erfindungsgemäßen Triisocyanat R¹ Methyl und R² Wasserstoff ist.

Ein weiterer Gegenstand der vorliegenden Erfindung ist ein Verfahren zur Herstellung des erfindungsgemäßen Triisocyanats, wobei ein 2-N, N-(Bis-2-aminoalkyl)-1,2-alkyldiaminderivat der Formel (I) mit Phosgen umgesetzt wird.

Bei den erfindungsgemäßen 2-N,N-(Bis-2-aminoalkyl)-1,2-alkyldiaminen der Formel (I) 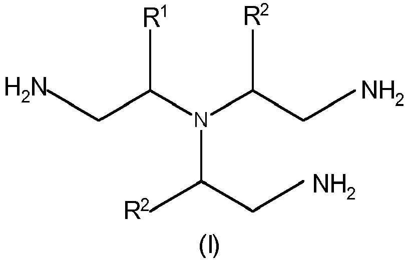 mit
- R¹: ausgewählt aus der Gruppe von verzweigten oder unverzweigten aliphatischen C₁₋₁₀-Alkylgruppen
- R²: ausgewählt aus der Gruppe von Wasserstoff und verzweigten oder unverzweigten aliphatischen C₁₋₁₀-Alkylgruppen,
ist R¹ bevorzugt ausgewählt aus der Gruppe von Methyl, Ethyl, n-Propyl, i-Propyl, n-Butyl, sec.-Butyl und tert.-Butyl, besonders bevorzugt sind Methyl und Ethyl insbesondere bevorzugt ist Methyl.

R² ist in der Formel (I) bevorzugt ausgewählt aus der Gruppe von Wasserstoff, Methyl, Ethyl, n-Propyl, i-Propyl, n-Butyl, sec.-Butyl und tert.-Butyl, besonders bevorzugt sind Wasserstoff, Methyl und Ethyl, insbesondere bevorzugt ist Wasserstoff.

Bevorzugte Verbindungen sind ausgewählt aus der Gruppe bei denen R¹ Methyl und R² Wasserstoff, R¹ Methyl und R² Methyl und R¹ Methyl und R² Ethyl ist, insbesondere bevorzugt ist die Verbindung bei der R¹ Methyl und R² Wasserstoff (N,N-(Bis-2-aminoethyl)-1,2-propandiamin (hier als MGTA bezeichnet)) ist.

Das erfindungsgemäße Verfahren zur Herstellung der Verbindungen der Formel (I) erfolgt im Allgemeinen durch Umsetzung der Verbindungen der Formel (II), 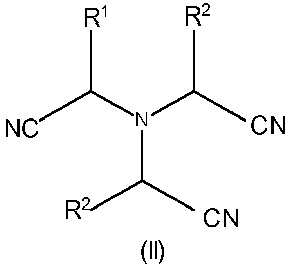 wobei R¹ und R² die obige Bedeutung haben mit Wasserstoff in Gegenwart eines Hydrierkatalysators. Bevorzugt wird das Hydrierverfahren, in dem Verbindungen der Formel (II) eingesetzt wird, ohne Ammoniak und kontinuierlich durchgeführt.

In einer besonderen Ausführungsform des obigen Verfahrens wird die Hydrierung in Abwesenheit von Ammoniak und als kontinuierliches Verfahren durchgeführt, wobei hier die Verbindungen der Formel (IIa) eingesetzt werden 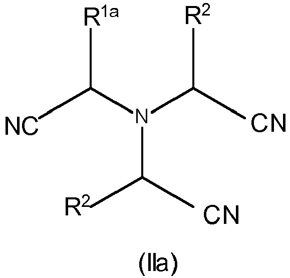 mit
- R^{1a}: ausgewählt aus der Gruppe von verzweigten oder unverzweigten aliphatischen C₁₋₁₀-Alkylgruppen und Wasserstoff und
- R²: ausgewählt aus der Gruppe von Wasserstoff und verzweigten oder unverzweigten aliphatischen C₁₋₁₀-Alkylgruppen.

Vorteilhaft ist jedoch das Verfahren in dem die Verbindungen der Formel (II) eingesetzt werden.

Die erfindungsgemäßen 2-N, N-(bis-2-aminoalkyl)-1,2-alkyldiamin-Derivate der Formel (I) können in vorteilhafter Weise bei hohem Umsatz und/oder hoher Selektivität hergestellt werden. Nebenprodukte, wie beispielsweise das entsprechende 2-piperazin-1-yl-alkyl-1-amin-Derivat oder das 2-(2,6-Dialkyl)piperazin-1-yl-alkyl-1-amin-Derivat fallen nur in geringen Mengen an bzw. können durch Steuerung von Verfahrensparametern wie Druck, Temperatur oder Katalysator weiter verringert werden. Insbesondere die Hydrierung von MGDN unter Erhalt des entsprechenden N, N-(bis-2-aminoethyl)-1,2-propandiamins als solchem (MGTA) kann mit hoher Selektivität von vorzugsweise 60 %, besonders bevorzugt > 65 %, durchgeführt werden.

Bei der Hydrierung zum erfindungsgemäßen 2-N,N-(Bis-2-aminoalkyl)-1,2-alkyldiaminderivat werden im Allgemeinen mindestens sechs Mole Wasserstoff pro Mol N,N-(Bis-2-cyanoalkyl)1,2-alkyldinitrilen benötigt. Jedoch kann das Verfahren auch mit einem Überschuss an Wasserstoff durchgeführt werden.

Die Temperaturen, bei denen die Hydrierung durchgeführt wird, liegen in einem Bereich von 60 bis 180 °C, bevorzugt von 80 bis 140 °C, insbesondere bei 100 bis 130 °C.

Der bei der Hydrierung herrschende Druck liegt im Allgemeinen bei 40 bis 300 bar, bevorzugt bei 40 bis 240 bar, besonders bevorzugt bei 80 bis 200 bar.

In einer bevorzugten Ausführungsform werden die Verbindung der Formel (II) oder (IIa) mit einer Rate der Hydrierung zugeführt, die nicht größer ist als die Rate, mit der die Verbindung der Formel (II) oder (IIa) mit Wasserstoff bei der Hydrierung reagiert.

Die Zuführrate ist bevorzugt so einzustellen, dass Vollumsatz erreicht wird. Dieses wird durch Temperatur, Druck, Art der Verbindung der Formel (II) oder (IIa), Menge und Art des Katalysators, des Reaktionsmediums, Durchmischungsgüte des Reaktorinhalts, Verweilzeit etc. beeinflusst.

Das erfindungsgemäße Verfahren wird in Gegenwart eines Katalysators durchgeführt. Als Katalysatoren können prinzipiell alle dem Fachmann für eine Nitrilhydrierung bekannten Katalysatoren eingesetzt werden. Als Katalysatoren zur Hydrierung der drei Nitrilfunktionen zu den erfindungsgemäßen Verbindungen der Formel (II) oder (IIa) können somit beispielsweise Katalysatoren eingesetzt werden, die als aktive Spezies ein oder mehrere Elemente der 8. Nebengruppe des Periodensystems (Fe, Co, Ni, Ru, Rh, Pd, Os, Ir, Pt), bevorzugt Fe, Co, Ni, Ru oder Rh, besonders bevorzugt Co oder Ni enthalten.

Darin eingeschlossen sind so genannte oxidische Katalysatoren, die ein oder mehrere aktive Spezies in Form ihrer sauerstoffhaltigen Verbindungen enthalten und so genannte Skelett-Katalysatoren (auch als Raney®-Typ bezeichnet; nachfolgend auch: Raney-Katalysator), die durch Auslaugen (Aktivierung) einer Legierung aus hydrieraktivem Metall und einer weiteren Komponente (bevorzugt Al) erhalten werden. Die Katalysatoren können zusätzlich einen oder mehrere Promotoren enthalten.

In einer besonders bevorzugten Ausführungsform werden bei Hydrierung von Verbindungen der Formel (II) oder (IIa) Raney-Katalysatoren eingesetzt, bevorzugt Raney-Kobalt- oder Raney-Nickel-Katalysatoren und besonders bevorzugt ein Raney-Kobalt-Katalysator, der als Promotor mindestens eines der Elemente Mo, Cr oder Fe enthält. Der Raney-Kobalt-Katalysator ist also mit mindestens einem dieser Elemente dotiert.

Die Katalysatoren können als Vollkatalysatoren oder geträgert eingesetzt werden. Als Träger kommen bevorzugt Metalloxide wie Al₂O₃, SiO₂, ZrO₂, TiO₂, Gemische von Metalloxiden oder Kohlenstoff (Aktivkohlen, Ruße, Graphit) zur Anwendung.

Die oxidischen Katalysatoren werden vor dem Einsatz außerhalb des Reaktors oder im Reaktor durch Reduktion der Metalloxide in einem Wasserstoff-enthaltendem Gasstrom bei erhöhter Temperatur aktiviert. Wenn die Katalysatoren außerhalb des Reaktors reduziert werden, kann danach eine Passivierung durch einen Sauerstoffenthaltenden Gasstrom oder die Einbettung in ein inertes Material erfolgen, um eine unkontrollierte Oxidation an Luft zu vermeiden und einen sicheren Umgang zu ermöglichen. Als inertes Material können organische Lösungsmittel wie Alkohole aber auch Wasser oder ein Amin, bevorzugt das Reaktionsprodukt, verwendet werden. Eine Ausnahme bei der Aktivierung stellen die Skelett-Katalysatoren dar, die durch Laugung mit wässriger Base, wie z. B. in EP-A 1 209 146 beschrieben, aktiviert werden können.
Je nach durchgeführtem Verfahren (Suspensionshydrierung, Wirbelschichtverfahren, Festbetthydrierung) werden die Katalysatoren als Pulver, Splitt oder Formkörper (bevorzugt Extrudate oder Tabletten) eingesetzt.

Besonders bevorzugte Festbettkatalysatoren sind die in EP-A1 742 045 offenbarten Cobalt-Vollkontakte, dotiert mit Mn, P, und Alkalimetall (Li, Na, K, Rb, Cs). Die katalytisch aktive Masse dieser Katalysatoren besteht vor der Reduktion mit Wasserstoff aus 55 bis 98 Gew.-%, insbesondere 75 bis 95 Gew.-%, Cobalt, 0,2 bis 15 Gew.-% Phosphor, 0,2 bis 15 Gew.-% Mangan und 0,05 bis 5 Gew.-% Alkalimetall, insbesondere Natrium, jeweils berechnet als Oxid.

Weitere geeignete Katalysatoren sind die in EP-A 963 975 offenbarten Katalysatoren, deren katalytisch aktive Masse vor der Behandlung mit Wasserstoff 22 bis 40 Gew.-% ZrCO₂, 1 bis 30 Gew.-% sauerstoffhaltige Verbindungen des Kupfers, berechnet als CuO, 15 bis 50 Gew.-% sauerstoffhaltige Verbindungen des Nickels, berechnet als NiO, wobei das molare Ni : Cu-Verhältnis größer 1 ist, 15 bis 50 Gew.-% sauerstoffhaltige Verbindungen des Kobalts, berechnet als CoO, 0 bis 10 Gew.-% sauerstoffhaltige Verbindungen des Aluminiums und/oder Mangans, berechnet als Al₂O₃ bzw. MnO₂, und keine sauerstoffhaltigen Verbindungen des Molybdäns enthält, beispielsweise der in diesem Dokument offenbarte Katalysator A mit der Zusammensetzung 33 Gew.-% Zr, berechnet als ZrO₂, 28 Gew.-% Ni, berechnet als NiO, 1 1 Gew.-% Cu, berechnet als CuO und 28 Gew.-% Co, berechnet als CoO.

Weiterhin geeignet sind die in EP-A 696 572 offenbarten Katalysatoren, deren katalytisch aktive Masse vor der Reduktion mit Wasserstoff 20 bis 85 Gew.-% ZrO₂, 1 bis 30 Gew.-% sauerstoffhaltige Verbindungen des Kupfers, berechnet als CuO, 30 bis 70 Gew.-% sauerstoffhaltige Verbindungen des Nickels, berechnet als NiO, 0,1 bis 5 Gew.-% sauerstoffhaltige Verbindungen des Molybdäns, berechnet als MoO₃, und 0 bis 10 Gew.-% sauerstoffhaltige Verbindungen des Aluminiums und/oder Mangans, berechnet als Al₂O₃ bzw. MnO₂ enthält. Beispielsweise der in dieser Schrift konkret offenbarte Katalysator mit der Zusammensetzung 31 ,5 Gew.-% ZrO₂, 50 Gew.-% NiO, 17 Gew.-% CuO und 1 ,5 Gew.-% MoO₃. Ebenso geeignet sind die in WO-A-99/44984 beschriebenen Katalysatoren enthaltend (a) Eisen oder eine Verbindung auf der Basis von Eisen oder deren Gemische, (b) von 0,001 bis 0,3 Gew.-% bezogen auf (a) eines Promoters auf der Basis von 2, 3, 4 oder 5 Elementen ausgewählt aus der Gruppe Al, Si, Zr, Ti, V, (c) von 0 bis 0,3 Gew.-% bezogen auf (a) einer Verbindung auf der Basis eines Alkali- und/oder Erdalkalimetalls, sowie (d) von 0,001 bis 1 Gew.-% bezogen auf (a) Mangan.

Für Suspensionsverfahren werden bevorzugt Raney-Katalysatoren eingesetzt. Bei den Raney-Katalysatoren wird der aktive Katalysator als "Metallschwamm" aus einer binären Legierung (Nickel, Eisen, Kobalt, mit Aluminium oder Silicium) durch Herauslösen eines Partners mit Säure oder Lauge hergestellt. Reste des ursprünglichen Legierungspartners wirken oft synergetisch.

Die im erfindungsgemäßen Verfahren eingesetzten Raney-Katalysatoren werden bevorzugt ausgehend von einer Legierung aus Kobalt oder Nickel, besonders bevorzugt Kobalt, und einer weiteren Legierungskomponente, die in Alkalien löslich ist, hergestellt. Bei dieser löslichen Legierungskomponente wird bevorzugt Aluminium verwendet, es können aber auch andere Komponenten wie Zink und Silicium oder Gemische aus solchen Komponenten eingesetzt werden.

Zur Aktivierung des Raney-Katalysators wird die lösliche Legierungskomponente ganz oder teilweise mit Alkali extrahiert, wofür zum Beispiel wässrige Natronlauge verwendet werden kann. Der Katalysator kann danach zum Beispiel mit Wasser oder organischen Lösungsmittel gewaschen werden.

In dem Katalysator können einzelne oder mehrere weitere Elemente als Promotoren anwesend sein. Beispiele für Promotoren sind Metalle der Nebengruppen IB, VIB und/oder VIII des Periodensystems, wie Chrom, Eisen, Molybdän, Nickel, Kupfer usw.

Die Aktivierung der Katalysatoren durch Auslaugen der löslichen Komponente (typscherweise Aluminium) kann entweder im Reaktor selbst oder vor Einfüllen in den Reaktor erfolgen. Die voraktivierten Katalysatoren sind luftempfindlich und pyrophor und werden deshalb in der Regel unter einem Medium wie z. B. Wasser, einem organischen Lösungsmittel oder einem Stoff, der bei der erfindungsgemäßen Reaktion zugegen ist (Lösungsmittel, Edukt, Produkt) aufbewahrt und gehandhabt oder in eine organische Verbindung, die bei Raumtemperatur fest ist, eingebettet.

In einer bevorzugten Ausführungsform wird erfindungsgemäß ein Raney-Kobalt-Skelett-Katalysator eingesetzt, der aus einer Co/Al-Legierung durch Laugung mit wässriger Alkalimetallhydroxid-Lösung, z.B. Natronlauge, und nachfolgender Waschung mit Wasser erhalten wurde, und bevorzugt als Promotoren mindestens eines der Elemente Fe, Ni oder Cr enthält.

Solche Katalysatoren enthalten typischerweise neben Kobalt noch 1 - 30 Gew.-% Al, besonders 2 - 12 Gew.-% Al, ganz besonders 3 - 6 Gew.-% Al, 0 - 10 Gew.-% Cr, bei-sonders 0,1 - 7 Gew.-% Cr, ganz besonders 0,5 - 5 Gew.-% Cr, insbesondere 1,5 - 3,5 Gew.-% Cr, 0 - 10 Gew.-% Fe, besonders 0,1 - 3 Gew.-% Fe, ganz besonders 0,2 - 1 Gew.-% Fe, und/oder 0 - 10 Gew.-% Ni, besonders 0,1 - 7 Gew.-% Ni, ganz besonders 0,5 - 5 Gew. % Ni, insbesondere 1 - 4 Gew.-% Ni, wobei die Gewichtsangaben jeweils auf das Katalysatorgesamtgewicht bezogen sind.

Als Katalysator im erfindungsgemäßen Verfahren kann zum Beispiel vorteilhaft ein Kobalt-Skelett-Katalysator "Raney 2724" der Firma W. R. Grace & Co. eingesetzt werden. Dieser Katalysator weist folgende Zusammensetzung auf:
Al: 2-6 Gew.-%, Co: ≥ 86 Gew.-%, Fe: 0-1 Gew.-%, Ni: 1-4 Gew.-%, Cr: 1,5-3,5 Gew.-%.

Ebenfalls kann erfindungsgemäß ein Nickel-Skelett-Katalysator eingesetzt werden, der aus einer Ni/Al-Legierung durch Laugung mit wässriger Alkalimetallhydroxid-Lösung, z.B. Natronlauge, und nachfolgender Waschung mit Wasser erhalten wurde, und bevorzugt als Promotoren mindestens eines der Elemente Fe, Cr enthält.

Solche Katalysatoren enthalten typischerweise neben Nickel noch
1 - 30 Gew.-% Al, besonders 2 - 20 Gew.-% Al, ganz besonders 5 - 14 Gew.-% Al, 0 - 10 Gew.-% Cr, besonders 0,1 - 7 Gew.-% Cr, ganz besonders 1 - 4 Gew.-% Cr, und/oder 0 - 10 Gew.-% Fe, besonders 0,1 - 7 Gew.-% Fe, ganz besonders 1 - 4 Gew.-% Fe, wobei die Gewichtsangaben jeweils auf das Katalysatorgesamtgewicht bezogen sind.

Die Katalysatoren können gegebenenfalls bei nachlassender Aktivität und/oder Selektivität mit den dem Fachmann bekannten Methoden, wie zum Beispiel in WO 99/33561 und den darin zitierten Schriften veröffentlicht, regeneriert werden.

Die Regenerierung des Katalysators kann im eigentlichen Reaktor (in situ) oder am ausgebauten Katalysator (ex situ) durchgeführt werden. Bei Festbettverfahren wird bevorzugt in situ regeneriert, bei Suspensionsverfahren wird bevorzugt ein Teil des Katalysators kontinuierlich oder diskontinuierliche entnommen, ex situ regeneriert und zurückgeführt.

Das erfindungsgemäße Verfahren kann in Gegenwart eines Lösungsmittels durchgeführt werden. Als Lösungsmittel eignen sich prinzipiell alle dem Fachmann bekannten Lösungsmittel, wobei sich die Lösungsmittel vorzugsweise gegenüber den Verbindungen der Formel (II) oder (IIa) inert verhalten.

Mögliche Lösungsmittel sind organische Lösungsmittel, beispielsweise Amide, wie N-Methylpyrrolidon (NMP) und Dimethylformamid (DMF), aromatische und aliphatische Kohlenwasserstoffe, wie Toluol, Alkohole wie Methanol, Ethanol, Propanol, Isopropanol, Butanol, Isobutanol, sekundäres Butanol und tertiäres Butanol, Amine, wie EDA bzw. Ethylamine und Ammoniak, Ester, wie Essigsäuremethylester oder Essigsäureethylester und Ether, wie Diisopropylether, Düsobutylether, Glykoldimethylether, Diglykoldimethylether, Dioxan und Tetrahydrofuran (THF).

Vorzugsweise ist das Lösungsmittel ein Amid, ein aromatischer Kohlenwasserstoff, ein Alkohol, ein Amin, ein Ester oder ein Ether. Mehr bevorzugt werden im erfindungsgemäßen Verfahren Ether verwendet, noch mehr bevorzugt zyklische Ether und besonders bevorzugt Tetrahydrofuran.

Das Lösungsmittel wird normalerweise im Gewichtsverhältnis zu den eingesetzten Verbindungen der Formel (II) oder (IIa) von 0,1:1 bis 15:1 eingesetzt. Die Konzentration der Verbindungen der Formel (II) oder (IIa) in der Lösung, in der die Hydrierung durchgeführt wird, sollte so gewählt werden, dass eine geeignete Zuführrate bzw. Verweilzeit eingestellt werden kann. Es ist bevorzugt die Verbindungen der Formel (II) oder (IIa) zu 5 bis 50 Gew.-% mit dem Lösungsmittel zu vermischen. Bezogen auf die besonders bevorzugten Lösungsmittel Tetrahydrofuran ist es beispielsweise vorteilhaft, die Verbindungen der Formel (II) oder (IIa) zu 10 bis 40 Gew.-% bezogen auf das Lösungsmittel einzusetzen.

Die Umsetzung der Verbindungen der Formel (II) oder (IIa) mit Wasserstoff in Gegenwart von Katalysatoren kann in üblichen für die Katalyse geeigneten Reaktionsgefäßen in einer Festbett-, Wirbelschicht-, Suspensionsfahrweise kontinuierlich, semikontinuierlich oder diskontinuierlich durchgeführt werden. Zur Durchführung der Hydrierung eignen sich Reaktionsgefäße, in denen eine Kontaktierung der Verbindungen der Formel (II) oder (IIa) und des Katalysators mit dem Wasserstoff unter Druck möglich ist.

Die Hydrierung in Suspensionsfahrweise kann in einem Rührreaktor, Strahlschlaufenreaktor, Strahldüsenreaktor, Blasensäulenreaktor bzw. in einer Kaskade derartiger gleicher oder verschiedener Reaktoren durchgeführt werden.

Die Hydrierung an einem Festbettkatalysator findet bevorzugt in einem oder mehreren Rohrreaktoren aber auch Rohrbündelreaktoren statt.

Die Hydrierung der Nitrilgruppen findet unter Freisetzung von Wärme statt, die in der Regel abgeführt werden muss. Die Wärmeabfuhr kann durch eingebaute Wärmeüberträgerflächen, Kühlmäntel oder außen liegende Wärmeüberträger in einem Umlaufkreis um den Reaktor erfolgen. Der Hydrierreaktor bzw. eine Hydrierreaktorkaskade kann in geradem Durchgang gefahren werden. Alternativ ist auch eine Kreislauffahrweise möglich, bei der ein Teil des Reaktoraustrages an den Reaktoreingang zurückgeführt wird, bevorzugt ohne vorherige Aufarbeitung des Kreislaufstromes.

Insbesondere kann der Kreislaufstrom mittels eines externen Wärmeüberträgers auf einfache und kostengünstige Weise gekühlt und somit die Reaktionswärme abgeführt werden.

Der Reaktor kann auch adiabat betrieben werden. Bei adiabatem Betrieb des Reaktors kann der Temperaturanstieg im Reaktionsgemisch durch Abkühlung der Zuläufe oder durch Zufuhr von "kaltem" organischem Lösungsmittel begrenzt werden.

Da der Reaktor selbst dann nicht gekühlt werden muss, ist eine einfache und kostengünstige Bauform möglich. Eine Alternative stellt ein gekühlter Rohrbündelreaktor (nur im Fall des Festbetts) dar. Auch eine Kombination der beiden Fahrweisen ist denkbar. Hierbei wird bevorzugt ein Festbett- einem Suspensionsreaktor nachgeschaltet.

Der Katalysator kann in einem Festbett angeordnet (Festbettfahrweise) sein oder im Reaktionsgemisch suspendiert (Suspensionsfahrweise) sein.

In einer besonders bevorzugten Ausführungsform ist der Katalysator im zu hydrierenden Reaktionsgemisch suspendiert.

Die Absetzgeschwindigkeit des Hydrierkatalysators in dem gewählten Lösungsmittel sollte niedrig sein, was bedeutet, dass er sich nicht innerhalb von 10 Sekunden absetzt, damit der Katalysator gut in Suspension gehalten werden kann. Die Partikelgröße der eingesetzten Katalysatoren beträgt bei der Suspensionsfahrweise daher bevorzugt zwischen 0,1 und 500 µm, insbesondere 1 und 100 µm.

Wird die Hydrierung der Verbindungen der Formel (II) oder (IIa) in Suspensionsfahrweise kontinuierlich durchgeführt, so werden die Verbindungen der Formel (II) oder (IIa) bevorzugt kontinuierlich dem Reaktor zugeführt und aus dem Reaktor kontinuierlich ein Strom entfernt, der die Hydrierungsprodukte (N,N-(Bis-2-aminoalkyl)-1,2-alkyldiaminderivate) enthält.

Bei der diskontinuierlichen Suspensionsfahrweise ist eine semibatch Fahrweise mit Zufuhr der Verbindungen der Formel (II) oder (IIa) bevorzugt.

Die Menge an Katalysator beträgt bevorzugt 1 bis 60 Gew.-%, besonders bevorzugt 5 bis 40 Gew.-%, und ganz besonders bevorzugt 20 bis 30 Gew.-%, bezogen auf das gesamte Reaktionsgemisch.

Die Verweilzeit im Reaktor beträgt bei diskontinuierlicher Suspensions-Fahrweise bevorzugt 0,1 bis 6 Stunden, besonders bevorzugt 0,5 bis 2 Stunden.

Die Verweilzeit im Reaktor beträgt bei kontinuierlicher Suspensionsfahrweise bevorzugt 0,1 bis 6 Stunden, besonders bevorzugt 0,5 bis 2 Stunden.

Die Katalysatorbelastung beträgt bei der kontinuierlichen Suspensionsfahrweise bzw. beim Semi-Batch-Verfahren 0,1 bis 5 kg, bevorzugt 0,2 bis 2 kg, besonders bevorzugt 0,3 bis 1 kg an Verbindungen der Formel (II) oder (IIa) pro kg Katalysator und Stunde.

Wird die Reaktion in Suspensionsfahrweise in einem Rührreaktor durchgeführt, so liegt der Leistungseintrag über den Rührer bevorzugt bei 0,1 bis 100 KW pro m³.

Gebrauchter Katalysator kann durch Filtration, Zentrifugieren oder Querstromfiltration abgetrennt werden. Dabei kann es notwendig sein, Verluste an ursprünglicher Katalysatormenge durch Abrieb und/oder Desaktivierung durch Zugabe von frischem Katalysator auszugleichen.

Im Anschluss an die Hydrierung kann der Austrag aus der Hydrierung gegebenenfalls weiter aufgereinigt werden. Der Katalysator kann nach dem Fachmann bekannten Methoden abgetrennt werden. In der Regel wird nach Abtrennung des Katalysators der während der Hydrierung vorhandene Wasserstoff abgetrennt.

Die Abtrennung von Wasserstoff erfolgt bevorzugt durch Absenkung des Drucks, bei dem die Hydrierung durchgeführt wurde, auf einen Wert, bei dem Wasserstoff gasförmig ist, die anderen Komponenten im Reaktionsaustrag aber in der Flüssigphase vorliegen. Vorzugsweise wird der Reaktionsaustrag von einem Hydrierdruck von bevorzugt 60 bis 325 bar, besonders bevorzugt 100 bis 280 bar, und ganz besonders bevorzugt 170 bis 240 bar auf einen Druck von 5 bis 50 bar in einen Behälter entspannt. Am Kopf des Behälters werden Wasserstoff, gegebenenfalls Ammoniak, sowie gegebenenfalls geringe Menge an verdampften Leichtsiedern oder Lösungsmitteln, wie THF, erhalten.

Im Reaktionsaustrag gegebenenfalls vorhandene organische Lösungsmittel werden im Allgemeinen ebenfalls destillativ abgetrennt. Insbesondere können die erfindungsgemäßen N, N-(Bis-2-aminoalkyl)-1,2-alkyldiamin-Derivate nach dem Fachmann bekannten Methoden aus dem Reaktionsprodukt isoliert werden.

Die vorliegende Erfindung betrifft zudem die Verwendung von den Verbindungen der Formel (I) als Härter für Epoxidharze, als Zwischenprodukt bei der Herstellung von Triisocyanaten, als Starter bei der Herstellung von Polyetherolen und/oder als Monomer für die Polyamidherstellung.

Die Verbindungen der Formel (I) stellen alternative Härter für Epoxidharze dar, die neue Möglichkeiten bei der Formulierung und Verarbeitung von Epoxidharzen ermöglichen und zur Regulierung des Eigenschaftsspektrums von Epoxidharzen eingesetzt werden können. Die erfindungsgemäßen Verbindungen der Formel (I) zeigen als Härter für Epoxidharze schnelle Aushärtezeiten und hohe Glasübergangstemperaturen was sie für die Anwendung in Klebstoffen, Bodenbeschichtungen und resin transfer modling (RTM) Anwendungen besonders geeignet macht.

Die Verbindungen der Formel (I) können auch als Zwischenprodukt bei der Herstellung der entsprechenden Triisocyanate der nachstehend aufgeführten allgemeinen Formel (III) 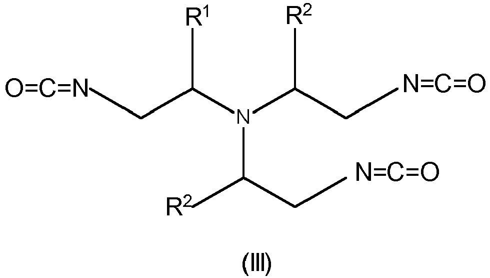 verwendet werden.

In den allgemeinen Formeln (III) haben die jeweiligen Reste wie R¹ und R² die gleiche Bedeutung wie vorstehend für die allgemeinen Formeln (I) definiert.

Diese Triisocyanate sind für die Herstellung von lichtbeständigen Polyurethanen, beispielsweise als Lack oder Beschichtung, geeignet und bieten aufgrund ihrer Struktur neue Formulierungsmöglichkeiten und damit Zugang zu neuen, interessanten Eigenschaftsprofilen. Diese Triisocyanate sind beispielsweise durch Umsetzung von Verbindungen der Formel (I) mit Phosgen erhältlich.

Die Verbindungen der Formel (I) können auch als Starter bei der Herstellung von Polyetherolen verwendet werden. Die Verbindungen der Formel (I) sind CH-acide Verbindungen, die mit einer Base deprotoniert werden können und an die nachfolgenden Alkylenoxide, wie Ethylenoxid, Propylenoxid und/oder Butylenoxid, addiert werden können.

Alkoxylierte Triamine können beispielsweise als Katalysatoren in der PUR-Herstellung eingesetzt werden.

Die Verbindungen der Formel (I) können als Monomer bei der Herstellung von Polyamiden eingesetzt werden. So können die Verbindungen der Formel (I) beispielsweise mit Dicarbonsäuren, wie beispielsweise Bernsteinsäure, Adipinsäure, Terephthalsäure und/oder Phthalsäure, zu Polymeren umgesetzt werden.

Ein weiterer Gegenstand der vorliegenden Erfindung ist somit auch ein Triisocyanat gemäß der allgemeinen Formel (III), 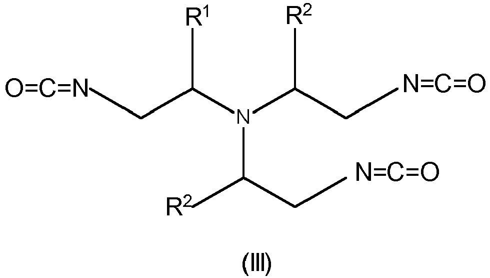 wobei R¹ und R² die gleiche Bedeutung wie in Formel (I) haben und auch die bevorzugten und besonders bevorzugte Varianten sind die gleichen wie für R¹ und R² aus Formel (I).

Ein weiterer Gegenstand der vorliegenden Erfindung ist somit auch ein Verfahren zur Herstellung eines Triisocyanats gemäß den vorstehend beschriebenen Definitionen.

Verfahren zur Herstellung von Triisocyanaten aus den entsprechenden Triaminen, beispielsweise durch Umsetzung mit Phosgen, sind dem Fachmann bekannt, so dass der Fachmann diese Verfahren entsprechend anwenden kann.

Erfindungsgemäß werden die Triisocyanate vorzugsweise hergestellt, indem eine Entsprechende Verbindung der Formel (I) mit Phosgen umgesetzt wird. Vorzugsweise wird dabei ein N, N-(Bis-2-aminoethyl)-1,2-propandiamin (MGTA) eingesetzt, gegebenenfalls können auch Gemische von zwei oder mehreren solcher Verbindungen der Formel (I) eingesetzt werden.

Nachfolgend wird die Erfindung anhand von Beispielen verdeutlicht.

### Beispiel 1

Herstellung von N,N-(bis-2-aminoethyl)-1,2-Propandiamin (Semi-Batch)

In einem 270 ml Autoklaven mit Strombrechern und Scheibenrührer wurden 5g (trocken) Raney-Cobalt und 40g THF vorgelegt. Der Autoklav wurde auf 120°C aufgeheizt und Wasserstoff bis zu einem Gesamtdruck von 100bar aufgepresst. Innerhalb von 2h wurde eine Mischung aus 10g reines MGDN in 90g THF zudosiert. Die Reaktionsmischung wurde weitere 60 Minuten unter Reaktionsbedingungen gerührt. Der Umsatz und die Selektivität wurden per GC Analytik (GC Säule:DB1, Länge =30m, Innendurchmesser = 0,32 mm, Filmdicke = 1 µm - 80°C Anfangstemperatur und Temperaturrampe 10°C/min auf 280°C - Trägergas =Helium - FID Detektor) bestimmt und in Flächen % angegeben.

Der Hydrieraustrag enthielt 61% N,N-(bis-2-aminoethyl)-1,2-propandiamin, 15% von den unten aufgeführten Piperazinderivaten der Formel (1), 7,5% von den unten aufgeführten Aminopipderivaten der Formel (2), 8% von den unten aufgeführten Dehydropipderivaten der Formel (3). Der Rest waren unbekannte Nebenkomponenten.

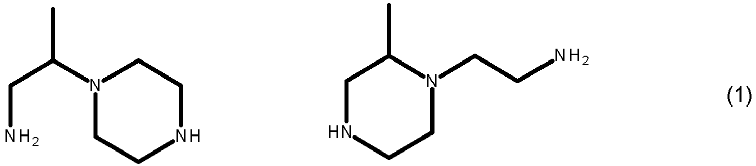

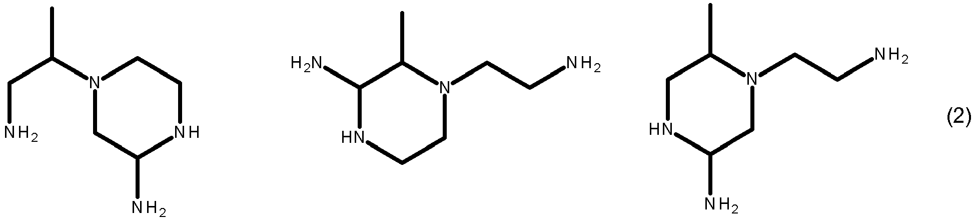

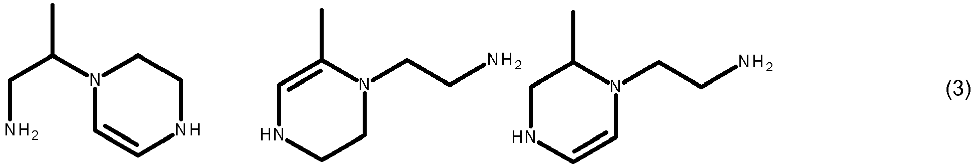

### Beispiel 2

### Herstellung von N,N-(bis-2-aminoethyl)-1,2-propandiamin (kontinuierlich)

In einem 270mL Autoklaven mit Stromstörern und Scheibenrührer wurden 7g (trocken) Raney-Cobalt und 0,1g 25% wässrige Natronlauge in 40g THF vorgelegt. Kontinuierlich wurden 15NL/h Wasserstoff zugefahren. 50g einer Mischung aus 10g reines MGDN in 90g THF wurden kontinuierlich pro Stunde bei 190bar zugepumpt. Die Temperatur im Reaktor betrug 120°C. Der Katalysator wurde durch kontinuierliche Filtration über eine Sintermetallfritte mit einem Porendurchmesser von 500 nm vom Reaktoraustrag getrennt. Der Austrag wurde über ein Regelventil entspannt. In einem nachgeschalteten Phasenscheider wurde anschließend Wasserstoff abgetrennt. Unter diesen Bedingungen wurde 100% Umsatz erreicht und die Ausbeute an N, N-(bis-2-aminoethyl)-1,2-propandiamin betrug 70% unter Einrechnung des Produktgehaltes in dem roh Austrag. Insgesamt wurden 300g MGDN eingesetzt und über diesen 60h Zeitraum blieb die Selektivität konstant. Neben dem erfindungsgemäßen Produkt (70% MGTA) enthielt der Hydrieraustrag 6% der oben stehenden Verbindungen der Formel (1), 5,5% der oben stehenden Verbindungen der Formel (2) und 4% der oben stehenden Verbindungen der Formel (3). Der Rest des Hydrieraustrages waren unbekannte Nebenkomponenten.
Ein Teil des roh Reaktionsgemisches wurde im Rotationsverdampfer eingeengt und über eine Vigreuxkolonne bei <0,5mbar destilliert. Bei 95°C wurde das Produkt über Kopf abdestilliert. Das Produkt N, N-(bis-2-aminoethyl)-1,2-propandiamin wurde mit einer Reinheit von 85% erhalten.
Das Produkt wurde durch GC-MS und NMR charakterisiert ¹³C-NMR (125 MHz, THF): 58.73, 53.73, 46.37, 41.85, 31.25. GC-MS : DB1 Säule, 30m, 0,32 mm, 1µm; 80°C Anfangstemperatur, Temperaturrampe 10°C/min auf 280°C - Retentionszeit 10,11 min (93,3 Fl.%). Die Bedingungen des GC-MS sind die gleichen wie bei Beispiel 1.

### Beispiel zur Verwendung in Epoxidharzsystemen

### Beispiel 3

Herstellung der Reaktionsharzmasse und Untersuchung des Reaktivitätsprofils Die miteinander zu vergleichenden Formulierungen wurden durch Mischen stöchiometrischer Mengen des Amins mit einem auf Bisphenol-A-diglycidylether basierenden Epoxidharz (EEW 182) hergestellt und sofort untersucht.
Die rheologischen Messungen zur Untersuchung des Reaktivitätsprofils der Amine mit Epoxidharzen wurden an einem schubspannungsgesteuerten Platte-Platte Rheometer (MCR 301, Anton Paar) mit einem Plattendurchmesser von 15 mm und einem Spaltabstand von 0.25 mm bei unterschiedlichen Temperaturen durchgeführt.
Untersuchung 3a) Vergleich der zu benötigenden Zeit, der frisch hergestellten Reaktionsharzmasse, um eine Viskosität von 10.000 mPa*s bei einer definierten Temperatur zu erreichen: die Messung wurde rotierend an dem oben genannten Rheometer bei verschiedenen Temperaturen (23°C, 75°C) durchgeführt.
Untersuchung 3b) Vergleich der Gelzeiten: die Messung wurde rotierend-oszillierend an dem oben genannten Rheometer bei 23°C und 75°C durchgeführt. Der Schnittpunkt von Verlustmodul (G") und Speichermodul (G') liefert die Gelzeit.

| **3** | **a** | | | | **b** | |
|---|---|---|---|---|---|---|
| | **Viskositätsanstieg auf 10.000 mPas** | | | | **Isotherme Gelzeit** | |
| | **23°C** | | **75°C** | | **23°C** | **75°C** |
| **Name** | **Startviskosität Ø 2-5 min [mPas]** | **Zeit [min]** | **Startviskosität [mPas]** | **Zeit [min]** | **Zeit [min]** | **Zeit [min]** |
| D230 | 886 | 478 | 39 | 40 | 1850 | 60 |
| T403 | 304 | 1269 | 60 | 39 | 2090 | 52 |
| TETA | 330 | 109 | 182 | 6.5 | 325 | 16 |
| MGTA | 620 | 72 | 206 | 6 | 195 | 10.5 |

Ergebnis
MGTA zeigt eine extrem kurze Gelzeit verglichen mit anderen aliphatischen Aminen Polyetheramin D230, Polyetheramin T403 und Triethylentetraamin (TETA)

### Beispiel 4

### Exothermes Profil der Reaktionsharzmasse und Glastemperaturen der ausgehärteten Duroplaste

Die DSC-Untersuchung der Härtungsreaktion der Amine mit einem auf Bisphenol-A-diglycidylether basierenden Epoxidharz (EEW 182) zur Bestimmung von Onset-Temperatur (Tₒ), Exothermie (ΔE) sowie die Bestimmung der Glastemperaturen (T_{g}) wurden nach ASTM D 3418 durchgeführt.

Untersuchung 4) Messprogramme für die DSC-Untersuchungen: 0°C → 5K/min 180°C → 30min 180°C → 20K/min 0°C → 20K/min 220°C.

| **4** | | | |
|---|---|---|---|
| **Name** | **Onset [°C]** | **ΔH [J/g]** | **Tg[°C]** |
| D230 | 83.3 | 421.9 | 93 |
| T403 | 84.3 | 399.3 | 90.3 |
| TETA | 63.1 | 586.8 | 151.6 |
| MGTA | 61.2 | 587.1 | 153.4 |

Ergebnis
MGTA zeigt eine extrem hohe Glasübergangstemperatur verglichen mit anderen aliphatischen Aminen.

## Patentansprüche

1. 2-N, N-(Bis-2-aminoalkyl)-1,2-alkyldiaminderivate der allgemeinen Formel (I) 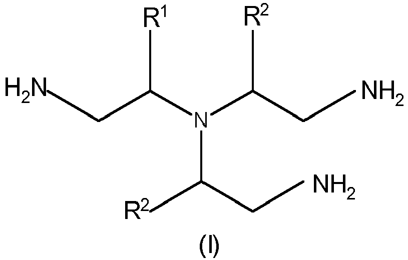 mit
R¹ ausgewählt aus der Gruppe von verzweigten oder unverzweigten aliphatischen C₁₋₁₀-Alkylgruppen
R² ausgewählt aus der Gruppe von Wasserstoff und verzweigten oder unverzweigten aliphatischen C₁₋₁₀-Alkylgruppen.

2. Die Verbindungen der Formel (I) gemäß Anspruch 1, wobei R¹ ausgewählt ist aus der Gruppe von Methyl, Ethyl, Propyl, Isopropyl, n-Butyl, sec.-Butyl und tert. Butyl und R² ausgewählt ist aus der Gruppe von Wasserstoff, Methyl, Ethyl, Propyl, Isopropyl, n-Butyl, sec.-Butyl und tert. Butyl.

3. Die Verbindungen der Formel (I) gemäß einem der Ansprüche 1 bis 2, wobei R¹ Methyl und R² Wasserstoff ist.

4. Verfahren zur Herstellung der Verbindungen der Formel (I) wobei die 2-N, N-(Bis-2-aminoalkyl)-1,2-alkyldiaminderivate aus dem entsprechenden 2-[N,N-(bis-1-cyanoalkyl)amino]alkylnitrile der Formel (II) 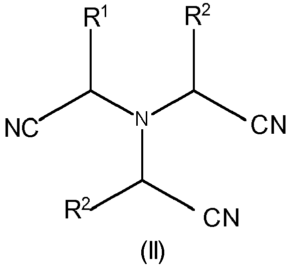 mit
R¹ ausgewählt aus der Gruppe von verzweigten oder unverzweigten aliphatischen C₁₋₁₀-Alkylgruppen
R² ausgewählt aus der Gruppe von Wasserstoff und verzweigten oder unverzweigten aliphatischen C₁₋₁₀-Alkylgruppen
durch Hydrierung in Gegenwart eines Katalysators und Wasserstoff hergestellt werden.

5. Verfahren nach Anspruch 4, wobei die Hydrierung kontinuierlich durchgeführt und unter Ausschluss von Ammoniak gearbeitet wird.

6. Verfahren zur Herstellung der Verbindungen der Formel (I) wobei die 2-N, N-(Bis-2-aminoalkyl)-1,2-alkyldiaminderivate aus den 2-[N,N-(bis-1-cyanoalkyl)amino]alkylnitrile der Formel (IIa) 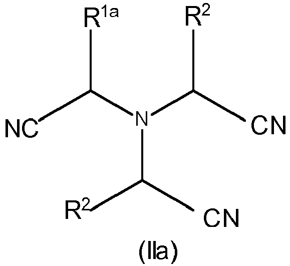 mit
R^{1a} ausgewählt aus der Gruppe von verzweigten oder unverzweigten aliphatischen C₁₋₁₀-Alkylgruppen und Wasserstoff
R² ausgewählt aus der Gruppe von Wasserstoff und verzweigten oder unverzweigten aliphatischen C₁₋₁₀-Alkylgruppen
durch Hydrierung in Gegenwart eines Katalysators und Wasserstoff hergestellt werden, wobei das Verfahren kontinuierlich und unter Ausschluss von Ammoniak durchgeführt wird.

7. Das Verfahren nach einem der Ansprüche 4 und 5 oder 6, wobei als Katalysator ein solcher eingesetzt wird der als aktive Spezies ein oder mehrere Elemente ausgewählt aus der Gruppe von Fe, Co, Ni, Ru, Rh, Pd, Os, Ir und Pt enthält.

8. Das Verfahren nach einem der Ansprüche 4,5 und 7 oder 6 und 7, wobei als Katalysator ein Raney-Nickel oder ein Raney-Kobalt-Katalysator eingesetzt wird.

9. Das Verfahren nach einem der Ansprüche 4, 5, 7 bis 8 oder 6 bis 8, wobei der eingesetzte Katalysator ein Raney-Kobalt-Katalysator ist, der als Promotor mindestens eins der Elemente Mo, Cr oder Fe enthält.

10. Das Verfahren nach einem der Ansprüche 4,5, 7 bis 9 oder 6 bis 9, wobei die Hydrierung in einem Lösungsmittel ausgewählt aus der Gruppe von Amiden, aromatischen Kohlenwasserstoffen, Alkoholen, Aminen, Estern und Ethern durchgeführt wird.

11. Das Verfahren nach einem der Ansprüche 4,5, 7 bis 10 oder 6 bis 10, wobei bei Temperaturen im Bereich von 60 bis 180°C und Drücken im Bereich von 40 bis 300 bar hydriert wird.

12. Das Verfahren nach einem der Ansprüche 4, 5, 7 bis 11 oder 6 bis 11, wobei Methylglycinnitril-diacetonitril (MGDN) zu N,N-(Bis-2-aminoethyl)-1 ,2-propandiamin (MGTA) hydriert wird.

13. Das Verfahren nach Anspruch 12, wobei Methylglycinnitril-diacetonitril (MGDN) in reiner kristalliner Form eingesetzt wird.

14. Verwendung eines der N,N-(Bis-2-aminoalkyl)-1,2-alkyldiaminderivate der Formel (I) nach einem der Ansprüche 1 bis 3 als Härter für Epoxyharze, als Zwischenprodukt bei der Herstellung von Triisocyanaten, als Starter bei Herstellung von Polyetherolen und/oder als Monomer für die Polyamidherstellung.

15. Triisocyanate gemäß der allgemeinen Formel (III) 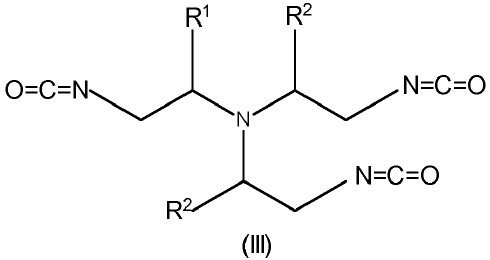 mit
R¹ ausgewählt aus der Gruppe von verzweigten oder unverzweigten aliphatischen C₁₋₁₀-Alkylgruppen und Wasserstoff
R² ausgewählt aus der Gruppe von Wasserstoff und verzweigten oder unverzweigten aliphatischen C₁₋₁₀-Alkylgruppen.

16. Triisocyanat gemäß Anspruch 15, wobei R¹ Methyl und R² Wasserstoff ist.

17. Verfahren zur Herstellung eines Triisocyanats nach einem der Ansprüche 15 oder 16, wobei ein 2-N, N-(Bis-2-aminoalkyl)-1,2-alkyldiaminderivat der Formel (I) nach Anspruch 1 oder 3 mit Phosgen umgesetzt wird.

## Claims

1. A 2-N,N-(bis-2-aminoalkyl)-1,2-alkyldiamine derivative of general formula (I) 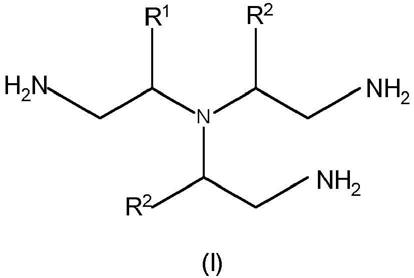 where
R¹ is selected from the group consisting of branched or unbranched aliphatic C₁₋₁₀ alkyl groups and
R² is selected from the group consisting of hydrogen and branched or unbranched aliphatic C₁₋₁₀ alkyl groups.

2. The compound of formula (I) according to claim 1, wherein R¹ is selected from the group consisting of methyl, ethyl, propyl, isopropyl, n-butyl, sec-butyl and tert-butyl and R² is selected from the group consisting of hydrogen, methyl, ethyl, propyl, isopropyl, n-butyl, sec-butyl and tert-butyl.

3. The compound of formula (I) according to claim 1 or claim 2, wherein R¹ is methyl and R² is hydrogen.

4. A process for preparing the compound of formula (I), wherein the 2-N,N-(bis-2-aminoalkyl)-1,2-alkyldiamine derivative is prepared from the corresponding 2-[N,N-(bis-1-cyanoalkyl)amino]alkylnitrile of formula (II) 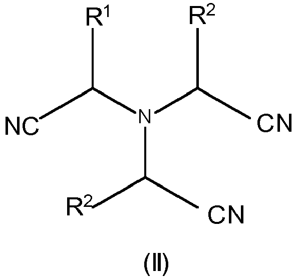 where
R¹ is selected from the group consisting of branched or unbranched aliphatic C₁₋₁₀ alkyl groups and
R² is selected from the group consisting of hydrogen and branched or unbranched aliphatic C₁₋₁₀ alkyl groups
by hydrogenation in the presence of a catalyst and hydrogen.

5. The process according to claim 4, wherein the hydrogenation is carried out as a continuous operation in the absence of ammonia.

6. A process for preparing the compound of formula (I), wherein the 2-N,N-(bis-2-aminoalkyl)-1,2-alkyldiamine derivative is prepared from the 2-[N,N-(bis-1-cyanoalkyl)amino]alkylnitrile of formula (IIa) 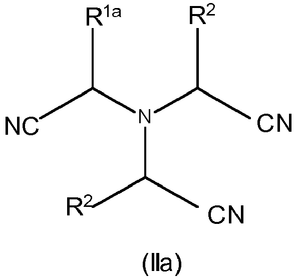 where
R^{1a} is selected from the group consisting of branched or unbranched aliphatic C₁₋₁₀ alkyl groups and hydrogen and
R² is selected from the group consisting of hydrogen and branched or unbranched aliphatic C₁₋₁₀ alkyl groups
by hydrogenation in the presence of a catalyst and hydrogen, wherein the process is carried out as a continuous operation in the absence of ammonia.

7. The process according to any one of claims 4 and 5 or 6, wherein a catalyst which comprises as active species one or more elements selected from the group consisting of Fe, Co, Ni, Ru, Rh, Pd, Os, Ir and Pt is used.

8. The process according to any one of claims 4, 5 and 7 or 6 and 7, wherein a Raney nickel catalyst or a Raney cobalt catalyst is used as the catalyst.

9. The process according to any one of claims 4, 5, 7 to 8 or 6 to 8, wherein the catalyst used is a Raney cobalt catalyst which comprises at least one of the elements Mo, Cr and Fe as promoter.

10. The process according to any one of claims 4, 5, 7 to 9 or 6 to 9, wherein the hydrogenation is carried out in a solvent selected from the group consisting of amides, aromatic hydrocarbons, alcohols, amines, esters and ethers.

11. The process according to any one of claims 4, 5, 7 to 10 or 6 to 10, wherein the hydrogenation is carried out at temperatures in the range from 60 to 180°C and at pressures in the range from 40 to 300 bar.

12. The process according to any one of claims 4, 5, 7 to 11 or 6 to 11, wherein methylglycinenitrile-N,N-diacetonitrile (MGDN) is hydrogenated to form N,N-bis(2-aminoethyl)-1,2-propanediamine (MGTA).

13. The process according to claim 12, wherein methylglycinenitrile-N,N-diacetonitrile (MGDN) is used in pure crystalline form.

14. The use of one of the N,N-(bis-2-aminoalkyl)-1,2-alkyldiamine derivatives of formula (I) according to any one of claims 1 to 3 as a hardener for epoxy resins, as an intermediate in the preparation of diisocyanates, as an initiator in the preparation of polyetherols, and/or as a monomer for the preparation of polyamides.

15. A triisocyanate according to general formula (III) 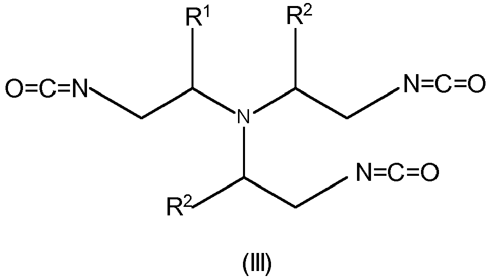 where
R¹ is selected from the group consisting of branched or unbranched aliphatic C₁₋₁₀ alkyl groups and hydrogen and
R² is selected from the group consisting of hydrogen and branched or unbranched aliphatic C₁₋₁₀ alkyl groups.

16. The triisocyanate according to claim 15, wherein R¹ is methyl and R² is hydrogen.

17. A process for preparing a triisocyanate according to claim 15 or 16, wherein a 2-N,N-(bis-2-aminoalkyl)-1,2-alkyldiamine derivative of formula (I) according to claim 1 or 3 is reacted with phosgene.

## Revendications

1. Dérivés de 2-N,N-(bis-2-aminoalkyl)-1,2-alkyldiamine de formule générale (I) 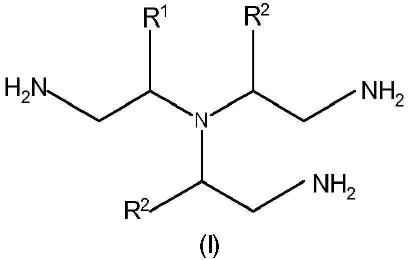 dans laquelle
R¹ est choisi dans le groupe formé par les groupes C₁₋₁₀-alkyle aliphatiques, ramifiés ou non ramifiés,
R² est choisi dans le groupe formé par hydrogène et les groupes C₁₋₁₀-alkyle aliphatiques, ramifiés ou non ramifiés,

2. Composés de formule (I) selon la revendication 1, R¹ étant choisi dans le groupe formé par méthyle, éthyle, propyle, isopropyle, n-butyle, sec-butyle et tert-butyle et R² étant choisi dans le groupe formé par hydrogène, méthyle, éthyle, propyle, isopropyle, n-butyle, sec-butyle et tert-butyle.

3. Composés de formule (I) selon l'une quelconque des revendications 1 à 2, R¹ représentant méthyle et R² représentant hydrogène.

4. Procédé pour la préparation des composés de formule (I), les dérivés de 2-N,N-(bis-2-aminoalkyl)-1,2-alkyldiamine étant préparés à partir des 2-[N,N-(bis-1-cyanoalkyl)amino]alkylnitriles correspondants de formule générale (II) 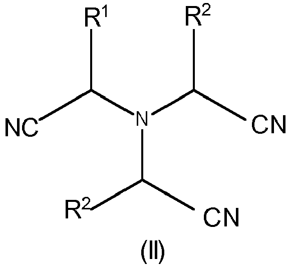 dans laquelle
R¹ est choisi dans le groupe formé par les groupes C₁₋₁₀-alkyle aliphatiques, ramifiés ou non ramifiés,
R² est choisi dans le groupe formé par hydrogène et les groupes C₁₋₁₀-alkyle aliphatiques, ramifiés ou non ramifiés,
par hydrogénation en présence d'un catalyseur et d'hydrogène.

5. Procédé selon la revendication 4, l'hydrogénation étant réalisée en continu et à l'abri d'ammoniac.

6. Procédé pour la préparation des composés de formule (I), les dérivés de 2-N,N-(bis-2-aminoalkyl)-1,2-alkyldiamine étant préparés à partir des 2-[N,N-(bis-1-cyanoalkyl)amino]alkylnitriles de formule (IIa) 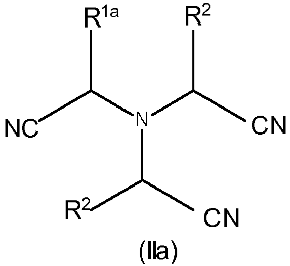 dans laquelle
R^{1a} est choisi dans le groupe formé par les groupes C₁₋₁₀-alkyle aliphatiques, ramifiés ou non ramifiés et hydrogène,
R² est choisi dans le groupe formé par hydrogène et les groupes C₁₋₁₀-alkyle aliphatiques, ramifiés ou non ramifiés,
par hydrogénation en présence d'un catalyseur et d'hydrogène, le procédé étant réalisé en continu et à l'abri d'ammoniac.

7. Procédé selon l'une quelconque des revendications 4 et 5 ou 6, le catalyseur qui est utilisé étant un catalyseur contenant, comme espèces actives, un ou plusieurs éléments choisis dans le groupe formé par Fe, Co, Ni, Ru, Rh, Pd, Os, Ir et Pt.

8. Procédé selon l'une quelconque des revendications 4, 5 et 7 ou 6 et 7, le catalyseur utilisé étant du nickel de Raney ou un catalyseur de type cobalt de Raney.

9. Procédé selon l'une quelconque des revendications 4, 5, 7 à 8 ou 6 à 8, le catalyseur utilisé étant un catalyseur de type cobalt de Raney, qui contient comme promoteur au moins un des éléments Mo, Cr ou Fe.

10. Procédé selon l'une quelconque des revendications 4, 5, 7 à 9 ou 6 à 9, l'hydrogénation étant réalisée dans un solvant choisi dans le groupe des amides, des hydrocarbures aromatiques, des alcools, des amines, des esters et des éthers.

11. Procédé selon l'une quelconque des revendications 4, 5, 7 à 10 ou 6 à 10, l'hydrogénation étant réalisée à des températures dans la plage de 60 à 180°C et à des pressions dans la plage de 40 à 300 bars.

12. Procédé selon l'une quelconque des revendications 4, 5, 7 à 11 ou 6 à 11, du méthylglycinenitrile-diacétonitrile (MGDN) étant hydrogéné en N,N-(bis-2-aminoéthyl)-1,2-propanediamine (MGTA).

13. Procédé selon la revendication 12, le méthylglycinenitrile-diacétonitrile (MGDN) étant utilisé sous forme cristalline pure.

14. Utilisation d'un des dérivés de N,N-(bis-2-aminoalkyl)-1,2-alkyldiamine de formule (I) selon l'une quelconque des revendications 1 à 3 comme durcisseur pour des résines époxy, en tant que produit intermédiaire lors de la préparation de triisocyanates, comme initiateur lors de la préparation de polyétherols et/ou comme monomère pour la préparation de polyamides.

15. Triisocyanates selon la formule générale (III) 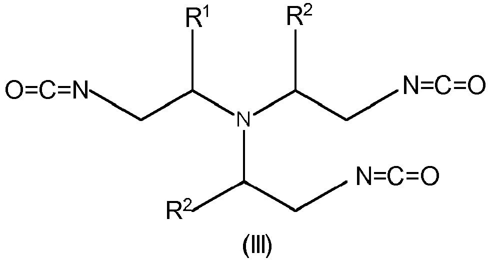 dans laquelle
R¹ est choisi dans le groupe formé par les groupes C₁₋₁₀-alkyle aliphatiques, ramifiés ou non ramifiés et hydrogène,
R² est choisi dans le groupe formé par hydrogène et les groupes C₁₋₁₀-alkyle aliphatiques, ramifiés ou non ramifiés,

16. Triisocyanate selon la revendication 15, R¹ représentant méthyle et R² représentant hydrogène.

17. Procédé pour la préparation d'un triisocyanate selon l'une quelconque des revendications 15 ou 16, un dérivé de 2-N,N-(bis-2-aminoalkyl)-1,2-alkyldiamine de formule (I) selon la revendication 1 ou 3 étant transformé avec du phosgène.
